# EUROPEAN PATENT APPLICATION

(11) **EP 3 881 777 A2**
(43) Date of publication of application: **22.09.2021**
(21) Application number: 21163374.8
(22) Date of filing: 18.03.2021
(51) Int. Cl.: A61B 17/072

(54) **SURGICAL BUTTRESSES FOR SURGICAL STAPLING APPARATUS**

(30) Priority: 19.03.2020 US 202062991652 P; 17.02.2021 US 202117177352
(71) Applicant: Covidien LP, Mansfield, MA 02048 (US)
(72) Inventor: MANDULA, Rajanikanth, 500040 Hyderabad (IN); GUTTI, Ravi Sekhar, 500049 Hyderabad (IN); FERNANDES, Roanit, 500026 Hyderabad (IN)
(74) Representative: Maschio & Soames IP Ltd

(57) **Abstract**

A loading unit includes a staple cartridge assembly, an anvil assembly, and an anvil buttress. The anvil assembly includes an anvil plate including a tissue facing surface having apertures defined therethrough and an anvil cover disposed over an outwardly facing surface of the anvil plate. The anvil buttress includes a body portion positioned against the tissue facing surface of the anvil plate and arms extending into the apertures of the anvil plate. The arms releasably secure the anvil buttress to the anvil plate.

## Description

### CROSS-REFERENCE TO RELATED APPLICATIONS

The present application claims the benefit of and priority to U.S. Provisional Patent Application Serial No. 62/991,652, filed on March 19, 2020, the entire content of which being hereby incorporated by reference.

### FIELD

The present disclosure is generally related to surgical stapling apparatus, and more particularly, to surgical buttresses and attachment assemblies for releasably securing the surgical buttresses to anvil assemblies of the surgical stapling apparatus.

### BACKGROUND

Surgical stapling apparatus are employed by surgeons to sequentially or simultaneously apply one or more rows of staples to body tissue for the purpose of joining segments of body tissue together. Such apparatus generally include a pair of jaws between which the body tissue to be joined is placed. When the surgical stapling apparatus is actuated, or "fired", longitudinally moving firing bars contact staple drive members in one of the jaws. The staple drive members push the staples through the body tissue and into an anvil in the opposite jaw which forms the staples. If body tissue is to be removed or separated, a knife blade can be provided in the jaws of the apparatus to cut the body tissue between the lines of staples.

Surgical supports, e.g., meshes or buttress materials, may be used in combination with surgical stapling apparatus to bridge, repair, and/or reinforce tissue defects within a patient. A clinician may manually attach the buttress materials to the surgical stapling apparatus in the operating room during a surgical procedure, or utilize a surgical stapling apparatus including buttress materials pre-installed thereon. The buttress material reinforces the staple or suture line as well as covers the juncture of the tissues to reduce leakage prior to healing.

### SUMMARY

The techniques of this disclosure generally relate to anvil buttress material attachment onto a loading unit of a surgical stapling apparatus. The anvil buttress attachment assemblies are designed to make buttress material attachment in the operating room a simple, straightforward, and cost effective procedure.

In one aspect, the disclosure provides a loading unit including a staple cartridge assembly, an anvil assembly, and an anvil buttress. The anvil assembly includes an anvil plate including a tissue facing surface having apertures defined therethrough and an anvil cover disposed over an outwardly facing surface of the anvil plate. The anvil buttress includes a body portion positioned against the tissue facing surface of the anvil plate and arms extending into the apertures of the anvil plate. The arms releasably secure the anvil buttress to the anvil plate.

Each of the apertures of the anvil plate may include a first opening in the tissue facing surface of the anvil plate and a second opening in the outwardly facing surface of the anvil plate. The apertures may taper from the tissue facing surface to the outwardly facing surface of the anvil plate such that the first opening in the tissue facing surface is larger than the second opening in the outwardly facing surface.

Each of the arms of the anvil buttress may extend into the first opening, through the aperture, and out the second opening of the aperture. Terminal end portions of the arms may be biased in a hooked configuration and extend out of the second opening of the aperture such that the terminal end portions grab onto the outwardly facing surface of the anvil plate.

The loading unit may further include a drive bar including an I-beam. The I-beam may include an anvil blade axially movable between the anvil plate and the anvil cover. The anvil blade may be in registration with portions of the arms of the anvil buttress extending out of the second opening of the apertures such that when the anvil blade is moved from a proximal position to a distal position, the anvil blade cuts the portions of the arms and frees the anvil buttress from the anvil assembly. The arms of the anvil buttress may extend from a first side of the body portion and be positioned inwardly of longitudinal edges of the body portion such that the arms are in the path of the anvil blade of the I-beam.

Each of the apertures of the anvil plate may include a cutout defined therein. Each of the arms of the anvil buttress may include a protrusion extending from an outer surface thereof that is received within the cutout of the aperture of the anvil plate to lock the arm to the anvil plate.

The body portion and the arms of the anvil buttress may be formed from biocompatible materials, and the arms may be stiffer than the body portion.

The buttress may further include a base layer positioned on a second side of the anvil buttress, and the arms of the anvil buttress may be coupled to the base layer and extend through the body portion and out a first side of the anvil buttress. The arms and the base layer of the anvil buttress may be stiffer than the body portion of the buttress.

The arms of the anvil buttress may be disposed in pairs on the proximal and distal end portions of the anvil buttress.

In another aspect, the disclosure provides an anvil buttress attachment assembly includes an anvil assembly and a buttress loading tray. The anvil buttress includes a body portion and arms extending from a first surface of the body portion. The buttress loading tray includes a cavity defined in a first side thereof that is sized and shaped to retain the body portion of the anvil buttress therein, and legs extending from a second surface of the buttress loading tray.

Terminal end portions of the arms of the anvil buttress may be disposed in a hooked configuration.

The arms of the anvil buttress may taper away from the body portion and include a protrusion extending from an outer surface thereof.

The arms of the anvil buttress may extend from proximal and distal end portions of the body portion and be positioned inwardly of longitudinal edges of the body portion.

In yet another aspect, the disclosure provides a method of releasably attaching an anvil buttress to a loading unit of a surgical stapling apparatus including: positioning a buttress loading tray onto a cartridge carrier of a staple cartridge assembly of a loading unit that does not contain a staple cartridge therein, the buttress loading tray defining a cavity retaining a body portion of an anvil buttress therein, the anvil buttress including arms extending from the body portion outwardly above the buttress loading tray; and approximating an anvil assembly and the staple cartridge assembly of the loading unit to a closed position such that the arms of the anvil buttress enter apertures defined in the anvil assembly and are retained therein to releasably secure the anvil buttress to the anvil assembly.

Positioning the buttress loading tray onto the cartridge carrier may include placing legs of the buttress loading tray over side walls of the cartridge carrier such that the anvil buttress extends across the cartridge carrier and is aligned with and opposed from the anvil assembly.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a perspective view of a surgical stapling apparatus in accordance with an embodiment of the present disclosure;
FIG. 2 is an exploded, perspective view of a tool assembly of the surgical stapling apparatus of FIG. 1 in accordance with an embodiment of the present disclosure;
FIG. 3 is a side, perspective view of the tool assembly of FIGS. 1 and 2, shown with anvil and cartridge buttresses attached to anvil and staple cartridge assemblies of the tool assembly;
FIG. 4 is a cross-sectional view of the anvil assembly of FIG. 3;
FIG. 5 is a cross-sectional view of the tool assembly of FIG. 3, with an anvil cover removed from the anvil assembly and a staple cartridge removed from the staple cartridge assembly, during firing of the surgical stapling apparatus;
FIG. 6 is a perspective view of an anvil buttress attachment assembly in accordance with an embodiment of the present disclosure, shown including the anvil buttress of FIG. 3;
FIG. 7 is a cross-sectional view of the anvil buttress attachment assembly of FIG. 6, taken along section line 7-7 of FIG. 6;
FIG. 8 is a cross-sectional view of the anvil buttress attachment assembly of FIG. 6 disposed on a cartridge carrier of the staple cartridge assembly of FIG. 3, shown with the tool assembly disposed in a closed position;
FIG. 9 is a close-up view of an anvil plate of the tool assembly and the anvil buttress attachment assembly of FIG. 8;
FIG. 10 is a side, perspective view of a tool assembly in accordance with another embodiment of the present disclosure, shown with anvil and cartridge buttresses attached to anvil and staple cartridge assemblies of the tool assembly;
FIG. 11 is a cross-sectional view of the anvil assembly of FIG. 10;
FIG. 12 is a perspective view of an anvil buttress attachment assembly in accordance with another embodiment of the present disclosure, shown including the anvil buttress of FIG. 10;
FIG. 13 is a cross-sectional view of the anvil buttress attachment assembly of FIG. 12, taken along section line 13-13 of FIG. 12;
FIG. 14 is a cross-sectional view of the anvil buttress attachment assembly of FIG. 12 disposed on a cartridge carrier of the staple cartridge assembly of FIG. 10, shown with the tool assembly disposed in a closed position; and
FIG. 15 is a close-up view of an anvil plate of the tool assembly and the anvil buttress attachment assembly of FIG. 14.

### DETAILED DESCRIPTION

Embodiments of the present disclosure will now be described in detail with reference to the drawing figures wherein like reference numerals identify similar or identical elements. Throughout this description, the term "proximal" refers to a portion of a structure, or component thereof, that is closer to a user, and the term "distal" refers to a portion of the structure, or component thereof, that is farther from the user.

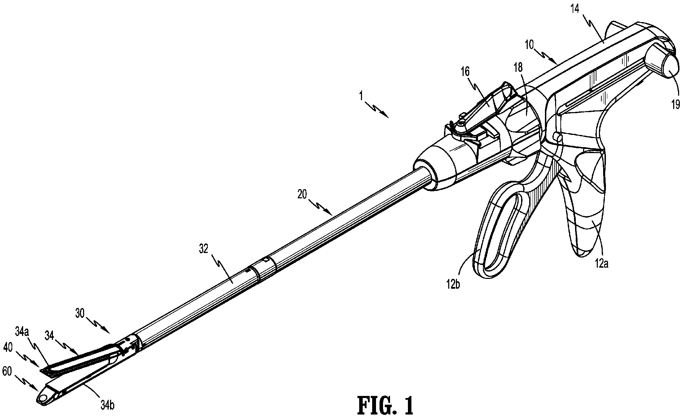

Referring now to FIG. 1, an exemplary surgical stapling apparatus or surgical stapler 1 is shown for use in stapling tissue in accordance with an embodiment of the present disclosure. The surgical stapling apparatus 1 generally includes a handle assembly 10, an elongate tubular body 20 extending distally from the handle assembly 10, and a loading unit 30 extending distally from the elongate tubular body 20. The loading unit 30 includes a housing portion 32 and a tool or jaw assembly 34 including first and second jaw members 34a, 34b. The first jaw member 34a and/or the second jaw members 34b is pivotable with respect to the housing portion 32 such that the tool assembly 34 is movable between an open position in which the first and second jaw members 34a, 34b are spaced apart with respect to each other, and a closed position in which the first and second jaw members 34a, 34b are substantially adjacent each other.

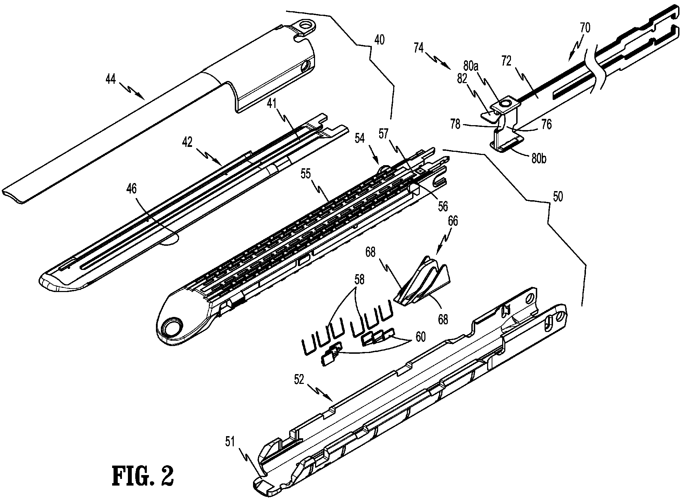

The handle assembly 10 includes a stationary handle member 12a, a movable handle member 12b, and a barrel portion 14. Actuation of the movable handle member 12b applies lines of staples 58 (FIG. 2) to tissue captured between the first and second jaw members 34a, 34b of the tool assembly 34. An articulation lever 16 is mounted on the forward end of the barrel portion 14 to facilitate articulation of the tool assembly 34. A rotatable member 18 is also mounted on the forward end of the barrel portion 14, adjacent the articulation lever 16. Rotation of the rotatable member 18 relative to the barrel portion 14 rotates the elongate tubular body 20 and the loading unit 30 relative to the handle assembly 10 so as to properly orient the tool assembly 34 relative to tissue to be stapled. A pair of knobs 19 is movably positionable along the barrel portion 14. The pair of knobs 19 is advanced distally to approximate or close the first and second jaw members 34a, 34b of the tool assembly 34 relative to each other, and retracted proximally to unapproximate or open the first and second jaw members 34a, 34b of the tool assembly 34 with respect to each other.

The loading unit 30 is a disposable loading unit ("DLU") that is releasably secured to the elongate tubular body 20 and thus, replaceable with a new loading unit 30. The loading unit 30 is a multi-use loading unit ("MULU") that is re-useable a predetermined number of times. For example, during a surgical procedure, the surgical stapling apparatus 1 can be used to staple and cut tissue, and a staple cartridge 54 (FIG. 2) of the MULU is replaced after each staple and cut operation of the surgical stapling apparatus 1 a pre-determined number of times before the entire MULU needs to be replaced. Alternatively, the loading unit 30 may be permanently affixed to the elongate tubular body 20.

As shown in FIGS. 1 and 2, the first jaw member 34a of the tool assembly 34 includes an anvil assembly 40 and the second jaw member 34b of the tool assembly 34 includes a staple cartridge assembly 50. The anvil assembly 40 includes an anvil plate 42 having a central longitudinal slot 41 formed therein, and a cover plate 44 secured over the anvil plate 42. The anvil plate 42 may include a plurality of staple forming pockets or cavities (not shown) defined in an inwardly or tissue facing surface thereof 46.

The staple cartridge assembly 50 includes a cartridge carrier 52 defining an elongated support channel 51 configured and dimensioned to selectively receive and support a staple cartridge 54 therein. The staple cartridge 54 may be removably and/or replaceably attached to the cartridge carrier 52 by, for example, a snap-fit connection, a detent, a latch, among other types of connectors within the purview of those skilled in the art. The staple cartridge 54 includes an inwardly or tissue facing surface 56 defining staple pockets or retention slots 55 formed therein for receiving a plurality of fasteners or staples 58 and staple pushers 60. A central longitudinal slot 57 is formed in and extends along a substantial length of the staple cartridge 54 which, together with the central longitudinal slot 41 of the anvil plate 42, facilitates passage of a knife blade 78 of a drive bar 70 therethrough.

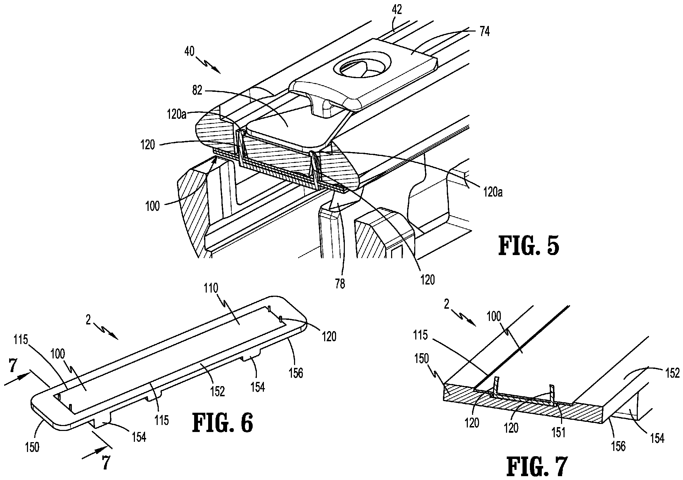

The drive bar 70 includes an elongated drive beam 72 configured to engage a drive member (not shown) of the elongate tubular body 20 (FIG. 1) of the surgical stapling apparatus 1 when the loading unit 30 is engaged therewith. The drive member imparts axial movement to the drive bar 70 from the handle assembly 10. An I-beam 74 is supported on a distal end of the drive beam 72 and is operatively associated with the tool assembly 34. The I-beam 74 includes a central wall portion 76 having the knife blade 78 defined in a distal edge thereof. The central wall portion 76 of the I-beam 74 is slidably disposed between the anvil and staple cartridge assemblies 40, 50, with upper and lower rails 80a, 80b of the I-beam 74, respectively, supported in the anvil and staple cartridge assemblies 40, 50. An anvil blade 82 extends distally from the upper rail 80a, above the knife blade 78, and is slidably disposed between the anvil plate 42 and the anvil cover 44 of the anvil assembly 40 (see e.g., FIG. 5). The anvil blade 82 is fan-shaped or shaped like a circular segment, and tapers outwardly away from the upper rail 80a. The anvil blade 82 is sharpened and configured to cut material disposed between the anvil plate 42 and the anvil cover 44, as described in further detail below.

During operation of the surgical stapling apparatus 1, distal advancement of the drive bar 70 causes an actuation sled 66 disposed within the staple cartridge assembly 50 to translate through the staple cartridge 54 and to advance cam wedges 68 of the actuation sled 66 into sequential contact with the staple pushers 60 which, in turn, cause the staple pushers 60 to translate vertically within the staple pockets 55 and urge the staples 58 from the staple pockets 55 towards the tissue facing surface 46 of the anvil plate 42 of the anvil assembly 40.

For a detailed description of the structure and function of exemplary surgical stapling apparatus, reference may be made to U.S. Patent Nos. 6,241,139, 6,330,965, and 7,819,896, the entire contents of each of which are incorporated herein by reference. It should be appreciated that principles of the present disclosure are equally applicable to surgical stapling apparatus having other configurations such as, for example, the types described in U.S. Patent Nos. 5,964,394, 7,128,253, and 7,334,717, the entire contents of each of which are incorporated herein by reference. Accordingly, it should be understood that a variety of surgical stapling apparatus may be utilized with the techniques of the present disclosure. For example, laparoscopic or open staplers, such as, for example, GIA™, Endo GIA™, TA™, and Endo TA™ staplers and/or linear and radial reloads with, for example, Tri-Staple™ technology, available through Medtronic (North Haven, CT) may be utilized with the anvil buttresses and anvil buttress attachment assemblies of the present disclosure.

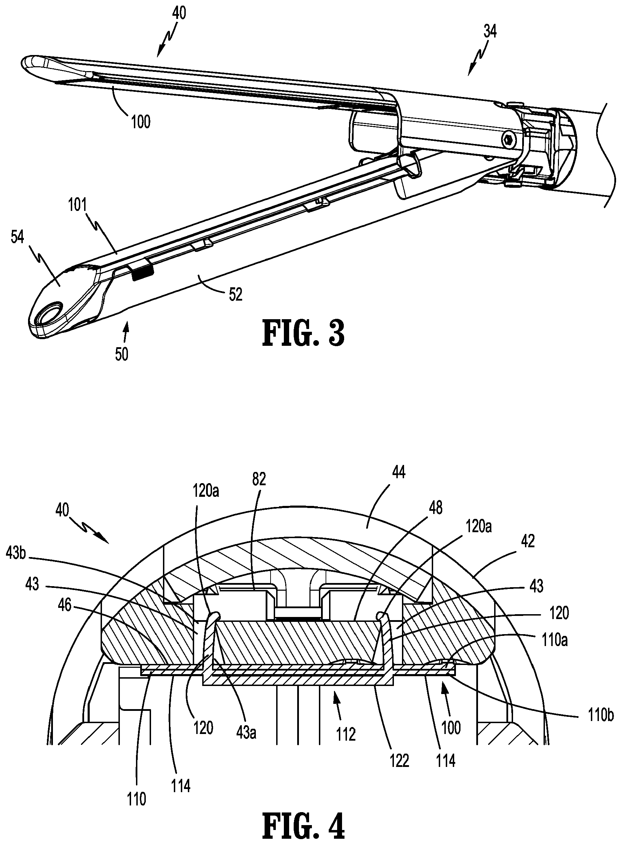

With reference now to FIG. 3, anvil and cartridge buttresses 100, 101 (also referred to herein generally as surgical buttresses or buttress materials) are shown releasably secured to the anvil and staple cartridge assemblies 40, 50, respectively, of the tool assembly 34. The surgical buttresses 100, 101 are fabricated from biocompatible materials which are bioabsorbable or non-absorbable, natural or synthetic materials. It should be understood that any combination of natural, synthetic, bioabsorbable, and/or non-bioabsorbable materials may be used to form the surgical buttresses 100, 101.

The surgical buttresses 100, 101 may be porous, non-porous, or combinations thereof. Suitable porous structures include, for example, fibrous structures (e.g., knitted structures, woven structures, and non-woven structures) and/or foams (e.g., open or closed cell foams). Suitable non-porous structures include, for example, films. The surgical buttresses 100, 101 described herein may be a single porous or non-porous layer, or include a plurality of layers including any combination of porous and non-porous layers. For example, the surgical buttresses 100, 101 may include multiple porous and non-porous layers that are stacked in an alternating manner. In another example, the surgical buttresses 100, 101 may be formed in a "sandwich-like" manner wherein the outer layers of the surgical buttress 100, 101 are porous and the inner layer(s) are non-porous, or vice versa. The anvil and cartridge buttresses 100, 101 may have the same or a different structure of layer(s).

Porous layer(s) in a surgical buttress may enhance the ability of the surgical buttress to absorb fluid, reduce bleeding, and/or seal a wound. Also, the porous layer(s) may allow for tissue ingrowth to fix the surgical buttress in place. Non-porous layer(s) in a surgical buttress may enhance the ability of the surgical buttress to resist tears and perforations during the manufacturing, shipping, handling, and/or stapling processes. Also, non-porous layer(s) may retard or prevent tissue ingrowth from surrounding tissues thereby acting as an adhesion barrier and preventing the formation of unwanted scar tissue.

With continued reference to FIG. 3, the cartridge buttress 101 is releasably secured to the staple cartridge 54 via any suitable attachment feature within the purview of those skilled in the art, such as, for example, mechanical attachment features (e.g., sutures, pins), chemical attachment features (e.g., adhesive), and/or attachment methods (e.g., welding). In embodiments, the cartridge buttress 101 is pre-loaded (e.g., by the manufacturer) onto the staple cartridge 54 which is then loadable into the cartridge carrier 52 of the staple cartridge assembly 50.

As shown in FIGS. 3 and 4, the anvil buttress 100 is releasably secured to the anvil assembly 40 by arms 120 of the anvil buttress 100 that extend into and engage the anvil plate 42. The anvil buttress 100 includes a body portion 110 having a generally rectangular shape corresponding to the tissue facing surface 46 of the anvil plate 42. The arms 120 of the anvil buttress 100 are coupled to the body portion 110 and extend outwardly away from a first side 110a of the body portion 110.

The arms 120 are fabricated from biocompatible materials as described above with respect to the anvil buttress 100, and may be formed from the same or different materials as the body portion 110 of the anvil buttress 100. In embodiments, the body portion 110 and the arms 120 of the anvil buttress 100 are formed from the same material, with the arms 120 being treated (e.g., crosslinked or processed with a stiffening agent) to be stiffer or more rigid than the body portion 110 so that the arms 120 can mechanically engage the anvil assembly 40. In embodiments, the arms 120 extend through the body portion 110 and are coupled to a base layer 122 positioned on a second side 110b of the body portion 110. The base layer 122 may have the same stiffness and/or rigidity as the arms 120 to support and strengthen the arms 120. In such embodiments, a central region 112 of the body portion 110 of the anvil buttress 100 is thickened as compared to longitudinally extending side regions 114 of the body portion 110 by the base layer 122.

The arms 120 are biased to extend at an angle away from body portion 110 (e.g., substantially orthogonally with respect to the body portion 110) such that when the body portion 110 of the anvil buttress 100 is positioned against the tissue facing surface 46 of the anvil assembly 40, the arms 120, which are aligned or in registration with apertures 43 defined through the anvil plate 42 extend into the apertures 43 and are secured therein. Terminal end portions 120a of the arms 120 are biased to extend inwardly (e.g., are bent or curved) towards the body portion 110 of the anvil buttress 100 thereby forming a hook or hooked configuration for grasping onto the anvil plate 42. As seen in FIG. 4, each of the apertures 43 tapers from the tissue contact surface 46 of the anvil plate 42 towards an outwardly facing surface 48 of the anvil plate 42 to define a larger opening 43a into the apertures 43 at the tissue facing surface 46 of the anvil plate and a smaller opening 43b of the apertures 43 at the outwardly facing surface 48 of the anvil plate 42. Thus, when the arms 120 are positioned through the apertures 43, the arms 120 bend inwardly due to the bias of the arms 120 and the shape of the apertures 43 such that the terminal end portions 120a of the arms 120 grab or lock onto the outwardly facing surface 48 of the anvil plate 42 thereby securing the anvil buttress 100 to the anvil assembly 40.

In operation, with the loading unit 30 loaded with both the anvil and cartridge buttresses 100, 101, as shown in FIG. 3, the surgical stapling apparatus 1 is used in accordance with methods known by those skilled in the art. Once the anvil and staple cartridge assemblies 40, 50 are approximated and clamped onto tissue, the surgical stapling apparatus 1 is fired, thereby stapling the anvil and cartridge buttresses 100, 101 to the tissue.

During firing, the knife blade 78 of the I-beam 74 travels distally through the tool assembly 34 and substantially simultaneously cuts and divides the tissue and the surgical buttresses 100, 101 disposed between the rows of formed staples 58. As the arms 120 of the anvil buttress 100 extend into the path of the anvil blade 82, as shown in FIG. 5, the anvil blade 82 cuts the terminal end portions 120a of the arms 120 extending between the anvil plate 42 and the anvil cover 44 (FIG. 4) thereby freeing the anvil buttress 100 from the anvil assembly 40. When firing is complete and the anvil and staple cartridge assemblies 40, 50 are opened and unclamped, the anvil and cartridge buttresses 100, 101, which are now stapled to the tissue, pull away from the anvil and staple cartridge assemblies 40, 50, and the tool assembly 34 can be removed from the surgical site.

FIGS. 6 and 7 illustrate an anvil buttress attachment assembly 2 including the anvil buttress 100 positioned within a buttress loading tray 150. The buttress loading tray 150 includes a cavity 151 defined in a first surface 152 thereof that corresponds in size and shape with the body portion 110 of the anvil buttress 100 such that the arms 120 of the anvil buttress 100 extend outwardly above the buttress loading tray 150. The arms 120 are provided in pairs at both proximal and distal end portions 100a, 100b of the anvil buttress 100 to secure the anvil buttress 100 to the anvil assembly 40. Additional arms 120 may be provided along the length of the body portion 110, and additional apertures 43 may be defined in the anvil plate 42 to correspond with the additional arms 120. The arms 120 are positioned inwardly of longitudinal edges 115 of the body portion 110 such that the arms 120 are in the path of the anvil blade 82 (FIG. 5) of the I-beam 74, as described above.

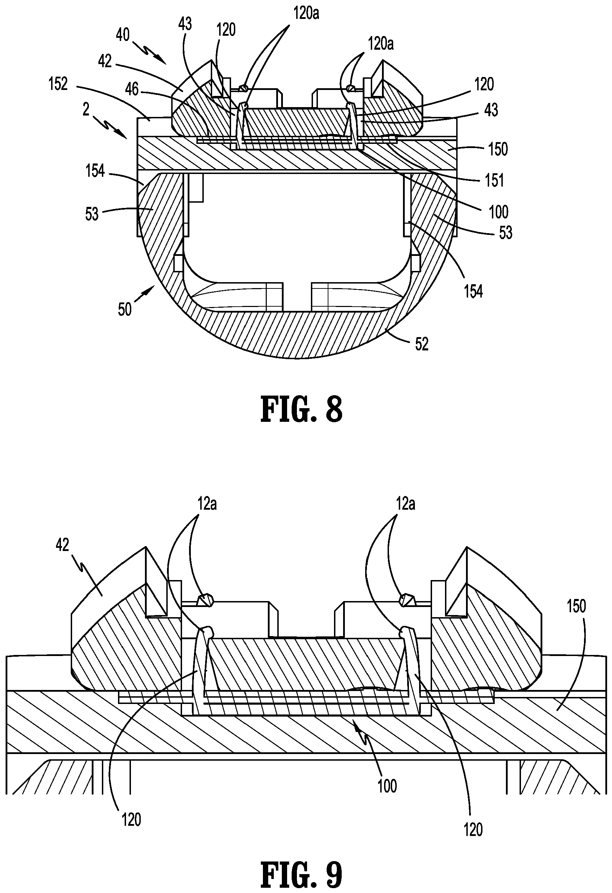

The buttress loading tray 150 includes legs 154 extending from a second surface 156 that are configured to engage side walls 53 (FIG. 8) of the cartridge carrier 52 of the staple cartridge assembly 50. Each of the legs 154 is bifurcated to receive the respective side wall 53 of the cartridge carrier 52 therein such that the anvil buttress 100 extends across the cartridge carrier 52 and is opposed from the tissue facing surface 46 of the anvil plate 42.

In a method of loading the anvil buttress 100 onto the anvil assembly 40 of the loading unit 30, the anvil buttress attachment assembly 2 is positioned on the cartridge carrier 52 of the staple cartridge assembly 50 prior to loading the cartridge carrier 52 with a staple cartridge 54 (e.g., the cartridge carrier 52 is empty and does not contain a staple cartridge 54 therein). The legs 154 of the buttress loading tray 150 of the anvil buttress attachment assembly 2 are aligned with the side walls 53 of the cartridge carrier 52 and the buttress loading tray 150 is pushed down onto the cartridge carrier 52 such that the first surface 152 of the buttress loading tray 150, which includes the anvil buttress 100 loaded into the cavity 151 thereof, faces the tissue facing surface 46 of the anvil assembly 40.

With the buttress loading tray 150 positioned on the cartridge carrier 52 of the staple cartridge assembly 50, the anvil and staple cartridge assemblies 40, 50 are approximated to the closed position (e.g., by pressing the handle assembly 10 of the surgical stapling apparatus 10) to bring the anvil assembly 40 into contact with the buttress loading tray 150, as shown in FIG. 8. As the anvil and staple cartridge assemblies 40, 50 are approximated, the arms 120 of the anvil buttress 100 enter the apertures 43 of the anvil plate 42 until the terminal end portions 120a of the arms 120 exit the apertures 43 and bend over the anvil plate 42 due to the bias of the arms 120 and/or the shape of the apertures 43 thereby securing the anvil buttress 100 to the anvil assembly 40, as seen in FIGS. 8 and 9. The anvil and staple cartridge assemblies 40, 50 are then opened and the buttress loading tray 150 is removed from the staple cartridge assembly 50. The staple cartridge assembly 50 is then loaded with a staple cartridge 54 (e.g., the staple cartridge 54 is pre-loaded with the cartridge buttress 100) making the surgical stapling apparatus 1 ready for a stapling operation.

After performing the stapling operation, the used staple cartridge 54 may then be removed from the staple cartridge assembly 50 of the tool assembly 34. In embodiments, additional or replacement staple cartridges 54, which may be pre-loaded with cartridge buttresses 101, may be secured to the loading unit 30, as needed or desired, and an anvil buttress 100 may be secured to the loading unit 30, as described above, prior to loading the staple cartridge 54 into the cartridge carrier 52 of the staple cartridge assembly 50.

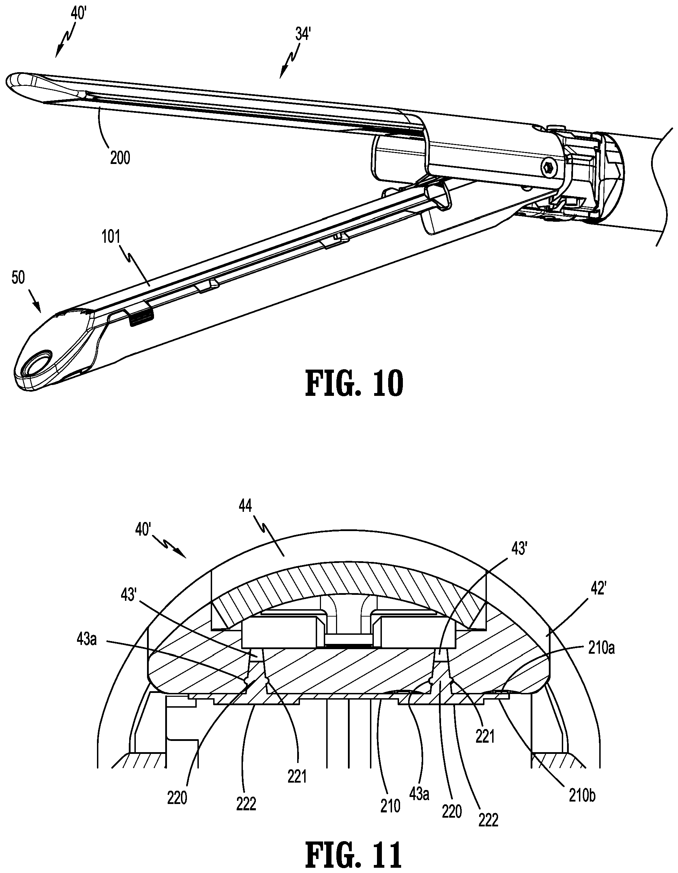

With reference now to FIG. 10, a tool assembly 34' is shown including an anvil buttress 200 releasably secured to an anvil assembly 40' of the tool assembly 34' and a cartridge buttress 101 releasably secured to a staple cartridge assembly 50 of the tool assembly 34'. It should be understood that various components of the tool assembly 34' of FIG. 10, with numbering followed by a prime symbol, correspond to components of the tool assembly 34 of FIG. 3, similarly numbered but not followed by a prime symbol, and various components of the anvil buttress 200, numbered in the 200 series, correspond to component of the anvil buttress 100, similarly numbered in the 100 series, such that redundant explanation of similar components of the embodiment of FIG. 10 to those of the embodiment of FIG. 3 need not be repeated herein. Accordingly, the tool assembly 34' and the anvil buttress 200 are substantially similar to the tool assembly 34 and the anvil buttress 100, and will be described with respect to the differences therebetween.

The anvil buttress 200 is releasably attached to the anvil assembly 40' by arms 220 of the anvil buttress 200 that extend into and engage the anvil plate 42' of the anvil assembly 40'. The arms 220 of the anvil buttress 200 extend perpendicularly from a first side 210a of the body portion 210 of the anvil buttress 200. The arms 220 extend through the body portion 210 and are coupled to base layers 222 positioned on a second side 210b of the body portion 210. The arms 220 have a tapered configuration that tapers away from the body portion 210. Each of the arms 220 includes a protrusion 221 extending from an outer surface of the arm 220 which is configured to engage and be received within a cutout or recess 43a defined in the aperture 43' of the anvil plate 42' such that when the anvil buttress 200 is loaded onto the anvil assembly 40', as seen in FIG. 11, the arms 220 lock into the anvil plate 42'.

In operation, with the tool assembly 34' loaded with both the anvil and cartridge buttresses 200, 101, as shown in FIG. 10, the surgical stapling apparatus 1 is used in a substantially similar manner as described above with regard to the tool assembly 34 of FIG. 3.

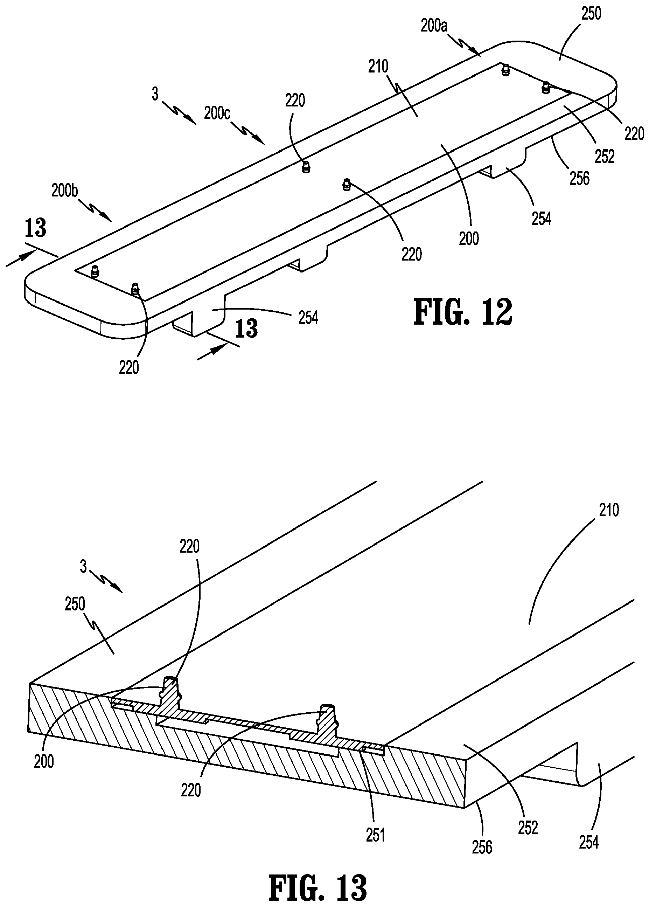

FIGS. 12 and 13 illustrate an anvil buttress attachment assembly 3 including the anvil buttress 200 positioned within a buttress loading tray 250. It should be understood that various components of the anvil buttress attachment assembly 3 of FIG. 12, numbered in the 200 series, correspond to components of the anvil buttress attachment assembly 2 of FIG. 6, similarly numbered in the 100 series, such that redundant explanation of similar components of the embodiment of FIG. 12 to those of the embodiment of FIG. 6 need not be repeated herein. Accordingly, the anvil buttress attachment assembly 3 is substantially similar to the anvil buttress attachment assembly 2, and will be described with respect to the differences therebetween.

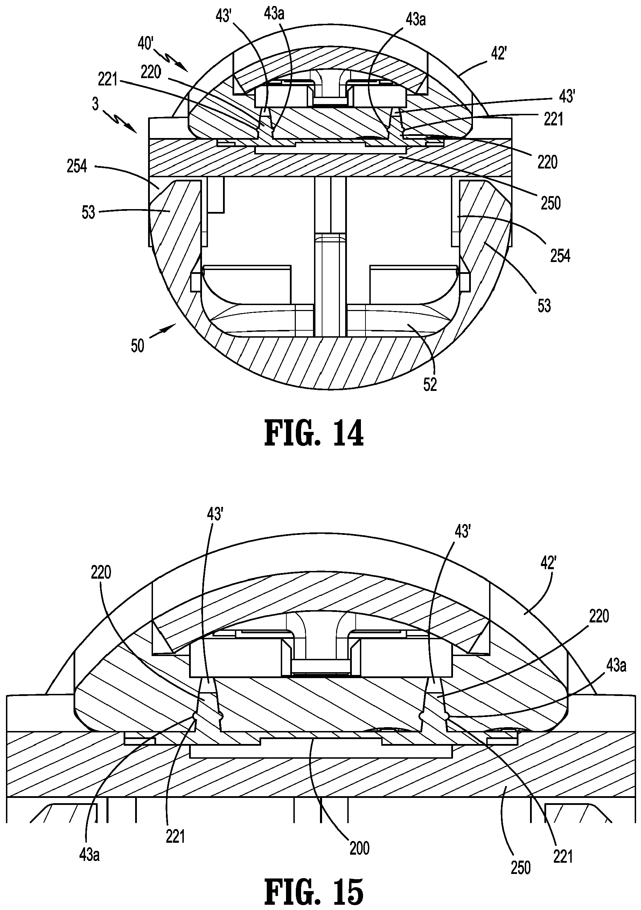

The buttress loading tray 250 includes a cavity 251 defined in a first surface 252 thereof that corresponds in size and shape with the body portion 210 of the anvil buttress 200 such that the arms 220 of the anvil buttress 200 extend outwardly above the buttress loading tray 250. The buttress loading tray 250 includes legs 254 extending from a second surface 256 that are configured to engage side walls 53 (FIG. 14) of the cartridge carrier 52 of the staple cartridge assembly 50. The arms 220 are provided in pairs at the proximal and distal end portions 200a, 200b, as well as the central portion 200c, of the anvil buttress 200 to secure the anvil buttress 200 to the anvil assembly 40'.

The anvil buttress 200 is loaded onto the anvil assembly 40' in the same manner as the anvil buttress 100 by positioning the anvil buttress attachment assembly 3 on the cartridge carrier 52 of the staple cartridge 50, as shown in FIG. 14. With the buttress loading tray 250 positioned on the cartridge carrier 52 of the staple cartridge assembly 50, the anvil and staple cartridge assemblies 40', 50 are approximated to the closed position such that the arms 220 of the anvil buttress 200 enter the apertures 43' of the anvil plate 42' until the protrusions 221 snap into the cutouts 43a of the apertures 43' thereby securing the anvil buttress 200 to the anvil assembly 40', as seen in FIGS. 14 and 15. The anvil and staple cartridge assemblies 40', 50 are then opened and the buttress loading tray 250 is removed from the staple cartridge assembly 50. The staple cartridge assembly 50 is then loaded with a staple cartridge 54 (e.g., the staple cartridge 54 is pre-loaded with the cartridge buttress 100) making the surgical stapling apparatus 1 ready for a stapling operation.

Although the anvil buttresses and anvil buttress attachment assemblies are discussed above for use with a surgical stapling apparatus having a manually operated, manually driven handle, any of the embodiments disclosed herein can include a surgical stapling apparatus having a hand-held powered handle having a motor, or a stapling unit that is attachable to a motorized drive, or a stapling unit arranged for use in a robotic surgical system. The anvil buttresses and anvil buttress attachment assemblies described herein may also be configured for use with other surgical apparatus, such as electromechanical surgical devices as described, for example, in U.S. Patent Appl. Pub. Nos. 2015/0157320 and 2015/0157321, the entire contents of each of which are incorporated herein by reference.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

While several embodiments of the disclosure have been shown in the drawings, it is not intended that the disclosure be limited thereto, as it is intended that the disclosure be as broad in scope as the art will allow and that the specification be read likewise. It is to be understood, therefore, that the present disclosure is not limited to the precise embodiments described, and that various other changes and modifications may be affected by one skilled in the art without departing from the scope or spirit of the disclosure. Additionally, the elements and features shown and described in connection with certain embodiments may be combined with the elements and features of certain other embodiments without departing from the scope of the present disclosure, and that such modifications and variation are also included within the scope of the present disclosure. Therefore, the above description should not be construed as limiting, but merely as exemplifications of preferred embodiments. Thus, the scope of the embodiments should be determined by the appended claims and their legal equivalents, rather than by the examples given.

The invention may be described by reference to the following numbered paragraphs: -
1. A loading unit comprising:
   a staple cartridge assembly;
   an anvil assembly including an anvil plate including a tissue facing surface having apertures defined therethrough and an anvil cover disposed over an outwardly facing surface of the anvil plate; and
   an anvil buttress including a body portion positioned against the tissue facing surface of the anvil plate and arms extending into the apertures of the anvil plate, the arms releasably securing the anvil buttress to the anvil plate.
2. The loading unit according to paragraph 1, wherein each of the apertures of the anvil plate include a first opening in the tissue facing surface of the anvil plate and a second opening in the outwardly facing surface of the anvil plate.
3. The loading unit according to paragraph 2, wherein the apertures taper from the tissue facing surface to the outwardly facing surface of the anvil plate such that the first opening in the tissue facing surface is larger than the second opening in the outwardly facing surface.
4. The loading unit according to paragraph 2, wherein each of the arms of the anvil buttress extends into the first opening, through the aperture, and out the second opening of the aperture.
5. The loading unit according to paragraph 4, wherein terminal end portions of the arms are biased in a hooked configuration and extend out of the second opening of the aperture such that the terminal end portions grab onto the outwardly facing surface of the anvil plate.
6. The loading unit according to paragraph 4, further comprising a drive bar including an I-beam, the I-beam including an anvil blade axially movable between the anvil plate and the anvil cover.
7. The loading unit according to paragraph 6, wherein the anvil blade is in registration with portions of the arms of the anvil buttress extending out of the second opening of the apertures such that when the anvil blade is moved from a proximal position to a distal position, the anvil blade cuts the portions of the arms and frees the anvil buttress from the anvil assembly.
8. The loading unit according to paragraph 6, wherein the arms of the anvil buttress extend from a first side of the body portion and are positioned inwardly of longitudinal edges of the body portion such that the arms are in the path of the anvil blade of the I-beam.
9. The loading unit according to paragraph 2, wherein each of the apertures of the anvil plate includes a cutout defined therein.
10. The loading unit according to paragraph 9, wherein each of the arms of the anvil buttress includes a protrusion extending from an outer surface thereof that is received within the cutout of the aperture of the anvil plate to lock the arm to the anvil plate.
11. The loading unit according to paragraph 1, wherein the body portion and the arms of the anvil buttress are formed from biocompatible materials, and the arms are stiffer than the body portion.
12. The loading unit according to paragraph 1, wherein the buttress further includes a base layer positioned on a second side of the anvil buttress, the arms of the anvil buttress are coupled to the base layer and extend through the body portion and out a first side of the anvil buttress.
13. The loading unit according to paragraph 12, wherein the arms and the base layer of the anvil buttress are stiffer than the body portion of the buttress.
14. The loading unit according to paragraph 1, wherein the arms are disposed in pairs on the proximal and distal end portions of the anvil buttress.
15. An anvil buttress attachment assembly comprising:
   an anvil buttress including a body portion and arms extending from a first surface of the body portion; and
   a buttress loading tray including a cavity defined in a first side thereof that is sized and shaped to retain the body portion of the anvil buttress therein, and legs extending from a second surface of the buttress loading tray.
16. The anvil buttress attachment assembly according to paragraph 15, wherein terminal end portions of the arms of the anvil buttress are disposed in a hooked configuration.
17. The anvil buttress attachment assembly according to paragraph 15, wherein the arms of the anvil buttress taper away from the body portion and include a protrusion extending from an outer surface thereof.
18. The anvil buttress attachment assembly according to paragraph 15, wherein the arms of the anvil buttress extend from proximal and distal end portions of the body portion and are positioned inwardly of longitudinal edges of the body portion.
19. A method of releasably attaching an anvil buttress to a loading unit of a surgical stapling apparatus, comprising:
   positioning a buttress loading tray onto a cartridge carrier of a staple cartridge assembly of a loading unit that does not contain a staple cartridge therein, the buttress loading tray defining a cavity retaining a body portion of an anvil buttress therein, the anvil buttress including arms extending from the body portion outwardly above the buttress loading tray; and
   approximating an anvil assembly and the staple cartridge assembly of the loading unit to a closed position such that the arms of the anvil buttress enter apertures defined in the anvil assembly and are retained therein to releasably secure the anvil buttress to the anvil assembly.
20. The method according to paragraph 19, wherein positioning the buttress loading tray onto the cartridge carrier includes placing legs of the buttress loading tray over side walls of the cartridge carrier such that the anvil buttress extends across the cartridge carrier and is aligned with and opposed from the anvil assembly.

## Claims

1. A loading unit comprising:
a staple cartridge assembly;
an anvil assembly including an anvil plate including a tissue facing surface having apertures defined therethrough and an anvil cover disposed over an outwardly facing surface of the anvil plate; and
an anvil buttress including a body portion positioned against the tissue facing surface of the anvil plate and arms extending into the apertures of the anvil plate, the arms releasably securing the anvil buttress to the anvil plate.

2. The loading unit according to claim 1, wherein each of the apertures of the anvil plate include a first opening in the tissue facing surface of the anvil plate and a second opening in the outwardly facing surface of the anvil plate.

3. The loading unit according to claim 2, wherein the apertures taper from the tissue facing surface to the outwardly facing surface of the anvil plate such that the first opening in the tissue facing surface is larger than the second opening in the outwardly facing surface.

4. The loading unit according to claim 2 or claim 3, wherein each of the arms of the anvil buttress extends into the first opening, through the aperture, and out the second opening of the aperture; preferably wherein terminal end portions of the arms are biased in a hooked configuration and extend out of the second opening of the aperture such that the terminal end portions grab onto the outwardly facing surface of the anvil plate.

5. The loading unit according to claim 4, further comprising a drive bar including an I-beam, the I-beam including an anvil blade axially movable between the anvil plate and the anvil cover; preferably wherein the anvil blade is in registration with portions of the arms of the anvil buttress extending out of the second opening of the apertures such that when the anvil blade is moved from a proximal position to a distal position, the anvil blade cuts the portions of the arms and frees the anvil buttress from the anvil assembly.

6. The loading unit according to claim 5, wherein the arms of the anvil buttress extend from a first side of the body portion and are positioned inwardly of longitudinal edges of the body portion such that the arms are in the path of the anvil blade of the I-beam.

7. The loading unit according to claim 2, wherein each of the apertures of the anvil plate includes a cutout defined therein; preferably wherein each of the arms of the anvil buttress includes a protrusion extending from an outer surface thereof that is received within the cutout of the aperture of the anvil plate to lock the arm to the anvil plate.

8. The loading unit according to any preceding claim, wherein the body portion and the arms of the anvil buttress are formed from biocompatible materials, and the arms are stiffer than the body portion.

9. The loading unit according to any preceding claim, wherein the buttress further includes a base layer positioned on a second side of the anvil buttress, the arms of the anvil buttress are coupled to the base layer and extend through the body portion and out a first side of the anvil buttress.

10. The loading unit according to claim 9, wherein the arms and the base layer of the anvil buttress are stiffer than the body portion of the buttress.

11. The loading unit according to any preceding claim, wherein the arms are disposed in pairs on the proximal and distal end portions of the anvil buttress.

12. An anvil buttress attachment assembly comprising:
an anvil buttress including a body portion and arms extending from a first surface of the body portion; and
a buttress loading tray including a cavity defined in a first side thereof that is sized and shaped to retain the body portion of the anvil buttress therein, and legs extending from a second surface of the buttress loading tray.

13. The anvil buttress attachment assembly according to claim 12, wherein terminal end portions of the arms of the anvil buttress are disposed in a hooked configuration.

14. The anvil buttress attachment assembly according to claim 12 or claim 13, wherein the arms of the anvil buttress taper away from the body portion and include a protrusion extending from an outer surface thereof; preferably wherein the arms of the anvil buttress extend from proximal and distal end portions of the body portion and are positioned inwardly of longitudinal edges of the body portion.

15. A method of releasably attaching an anvil buttress to a loading unit of a surgical stapling apparatus, comprising:
positioning a buttress loading tray onto a cartridge carrier of a staple cartridge assembly of a loading unit that does not contain a staple cartridge therein, the buttress loading tray defining a cavity retaining a body portion of an anvil buttress therein, the anvil buttress including arms extending from the body portion outwardly above the buttress loading tray; and
approximating an anvil assembly and the staple cartridge assembly of the loading unit to a closed position such that the arms of the anvil buttress enter apertures defined in the anvil assembly and are retained therein to releasably secure the anvil buttress to the anvil assembly; preferably wherein positioning the buttress loading tray onto the cartridge carrier includes placing legs of the buttress loading tray over side walls of the cartridge carrier such that the anvil buttress extends across the cartridge carrier and is aligned with and opposed from the anvil assembly.
